# EUROPEAN PATENT APPLICATION

(11) **EP 4 210 070 A1**
(43) Date of publication of application: **12.07.2023**
(21) Application number: 22151001.9
(22) Date of filing: 11.01.2022
(51) Int. Cl.: G16H 30/40, G16H 40/67, G16H 50/20, G16H 50/70

(54) **MACHINE LEARNING BASED ON RADIOLOGY REPORT**

(71) Applicant: Siemens Healthcare GmbH, 91052 Erlangen (DE)
(72) Inventor: Chekkoury, Andrei, 91054 Erlangen (DE); Comaniciu, Dorin, Princeton 08540 (US); Eibenberger, Eva, 90427 Nürnberg (DE); Georgescu, Bogdan, Princeton 08540 (US); Gibson, Eli, Plainsboro 08536 (US); Soza, Grzegorz, 90562 Heroldsberg (DE); Sühling, Michael, 91052 Erlangen (DE)

(57) **Abstract**

Techniques of determining a performance of a trained machine-learning algorithm are disclosed. The trained ML algorithm can be configured to process medical imaging data and thereby generate a prediction of at least one diagnosis of a patient based on the medical imaging data. The prediction of the at least one diagnosis of the patient is compared with a validated label of the at least one diagnosis of the patient and thereby the performance of the trained ML algorithm is determined based on the comparison. The validated label of the at least one diagnosis of the patient is obtained by parsing a validated radiology report of the patient and the medical imaging data is associated with the validated radiology report. If the performance of the trained ML algorithm is lower than a pre-defined threshold, an update of parameters of the trained ML algorithm may be triggered based on the validated label.

## Description

Various examples of the disclosure relate to facilitating an assessment of a performance of a trained machine-learning algorithm. Various examples of the disclosure specifically relate to determining the performance of the trained machine-learning algorithm based on a comparison of a validated label of at least one diagnosis parsed from a validated radiology report and a prediction of the at least one diagnosis generated by the trained machine-learning algorithm.

The use of Artificial intelligence (AI) algorithms, such as machine-learning (ML) algorithms, within the healthcare field, is gaining more and more traction and these algorithms are establishing themselves into the clinical routine. The countless benefits of using these algorithms in the radiological field are indisputable. ML algorithms can be used, e.g., to predict a diagnosis based on medical imaging data. Thereby, a medical practitioner can be assisted.

According to reference techniques, the training of ML algorithms is based on using large numbers of annotated datasets aiming in generating robust and generalizable ML algorithms, such as deep neural networks (DNN).

Traditional training of ML algorithms cannot account for the vast heterogeneity of the clinical field, e.g. variations caused in input data, scan protocols, scanner types, demographics, etc. Consequently, once deployed, the trained ML algorithms may generate false or inaccurate results.

Currently, such false or inaccurate results are identified manually by clinical experts, such as doctors or radiology technicians, which is extremely time-consuming. Further, single case feedback, e.g., correct or false algorithm results, provided by the experts is coupled with considerable implementation and usage effort, as well as bias, e.g. correcting only false negatives, but not false positives.

Accordingly, there is a need for advanced techniques of assessing a performance of a trained ML algorithm. Specifically, there is a need for advanced techniques of determining the performance of the trained ML algorithm that processes medical imaging data.

This need is met by the features of the independent claims. The features of the dependent claims define embodiments.

Hereinafter, techniques of determining a performance of a trained ML algorithm will be described. The performance of the trained ML algorithm is determined based on a comparison of a validated label of at least one diagnosis parsed from a validated radiology report and a prediction of the at least one diagnosis generated by the trained ML algorithm. An update or retraining of the trained ML algorithm may be performed based on the validated label of the at least one diagnosis parsed from the validated radiology report.

A computer-implemented method is provided. The method comprises obtaining a validated radiology report of a patient and medical imaging data of the patient associated with the validated radiology report. The method further comprises parsing the validated radiology report to obtain a validated label of at least one diagnosis. The method further comprises generating, by a trained machine-learning algorithm and at a computing device, a prediction of the at least one diagnosis based on the medical imaging data. The method additionally comprises determining a performance of the trained machine-learning algorithm based on a comparison of the validated label of the at least one diagnosis and the prediction of the at least one diagnosis.

A computer program or a computer-program product or a computer-readable storage medium includes program code. The program code can be loaded and executed by at least one processor. Upon loading and executing the program code, the at least one processor performs a method. The method comprises obtaining a validated radiology report of a patient and medical imaging data of the patient associated with the validated radiology report. The method further comprises parsing the validated radiology report to obtain a validated label of at least one diagnosis. The method further comprises generating, by a trained machine-learning algorithm and at a computing device, a prediction of the at least one diagnosis based on the medical imaging data. The method additionally comprises determining a performance of the trained machine-learning algorithm based on a comparison of the validated label of the at least one diagnosis and the prediction of the at least one diagnosis.

A device includes at least one processor and at least one memory. The at least one processor is configured to load program code from the at least one memory and execute the program code. Upon executing the program code, the at least one processor performs a method. The method comprises obtaining a validated radiology report of a patient and medical imaging data of the patient associated with the validated radiology report. The method further comprises parsing the validated radiology report to obtain a validated label of at least one diagnosis. The method further comprises generating, by a trained machine-learning algorithm and at a computing device, a prediction of the at least one diagnosis based on the medical imaging data. The method additionally comprises determining a performance of the trained machine-learning algorithm based on a comparison of the validated label of the at least one diagnosis and the prediction of the at least one diagnosis.

The device can be implemented by using a processor comprised in a data processing unit to execute a computer program implementing the method. The data processing unit can e. g. comprise a work station, a server, a cloud-based solution or an embedded device that can e. g. be integrated into a medical imaging device.

Additionally, the invention concerns a computer program comprising instructions which, when the program is executed by a processor, cause the processor to carry out the inventive method.

Additionally, the invention concerns a computer-readable storage medium having stored thereon on a computer program according to the present invention.

In particular, the features and advantages described in connection with the computer implemented method according to the invention can also be designed as corresponding subunits of the device according to the invention or of a computer program according to the invention. Conversely, the features and advantages described in connection with the device according to the invention or a computer program according to the invention can also be designed as corresponding method steps of the method according to the invention.

It is to be understood that the features mentioned above and those yet to be explained below may be used not only in the respective combinations indicated, but also in other combinations or in isolation without departing from the scope of the invention.

### BRIEF DESCRIPTION OF THE DRAWINGS

FIG. 1 schematically illustrates details with respect to a system according to various examples.
FIG. 2 is a flowchart of a method according to various examples.
FIG. 3 is a block diagram of a device according to various examples.

### DETAILED DESCRIPTION OF THE DRAWINGS

Some examples of the present disclosure generally provide for a plurality of circuits or other electrical devices. All references to the circuits and other electrical devices and the functionality provided by each are not intended to be limited to encompassing only what is illustrated and described herein. While particular labels may be assigned to the various circuits or other electrical devices disclosed, such labels are not intended to limit the scope of operation for the circuits and the other electrical devices. Such circuits and other electrical devices may be combined with each other and/or separated in any manner based on the particular type of electrical implementation that is desired. It is recognized that any circuit or other electrical device disclosed herein may include any number of microcontrollers, a graphics processor unit (GPU), integrated circuits, memory devices (e.g., FLASH, random access memory (RAM), read only memory (ROM), electrically programmable read only memory (EPROM), electrically erasable programmable read only memory (EEPROM), or other suitable variants thereof), and software which co-act with one another to perform operation(s) disclosed herein. In addition, any one or more of the electrical devices may be configured to execute a program code that is embodied in a non-transitory computer readable medium programmed to perform any number of the functions as disclosed.

In the following, embodiments of the invention will be described in detail with reference to the accompanying drawings. It is to be understood that the following description of embodiments is not to be taken in a limiting sense. The scope of the invention is not intended to be limited by the embodiments described hereinafter or by the drawings, which are taken to be illustrative only.

The drawings are to be regarded as being schematic representations and elements illustrated in the drawings are not necessarily shown to scale. Rather, the various elements are represented such that their function and general purpose become apparent to a person skilled in the art. Any connection or coupling between functional blocks, devices, components, or other physical or functional units shown in the drawings or described herein may also be implemented by an indirect connection or coupling. A coupling between components may also be established over a wireless connection. Functional blocks may be implemented in hardware, firmware, software, or a combination thereof.

Various techniques disclosed herein generally relate to facilitating assessing/determining a performance of a trained ML algorithm. The trained ML algorithm can be configured to process medical imaging data and thereby generate a prediction of at least one diagnosis of a patient based on the medical imaging data. The prediction of the at least one diagnosis of the patient is compared with a validated label of the at least one diagnosis of the patient and thereby the performance of the trained ML algorithm is determined based on the comparison. The validated label of the at least one diagnosis of the patient is obtained by parsing a validated radiology report of the patient and the medical imaging data is associated with the validated radiology report.

For example, the trained ML algorithm could be configured to process medical imaging data, e.g., depicting an anatomical target region of a patient, e.g., the heart, the liver, the brain, etc.. In other examples, other kind of imaging data could be processed, e.g., projection imaging data, e.g., for security scanners or material inspection.

According to the disclosure, various kinds and types of medical imaging data may be processed. As a general rule, it would be possible that the trained ML algorithm processes 2-D images or raw data obtained in K-space. The trained ML algorithm may process 3-D depth data, e.g., point clouds or depth maps. Voxel data structures may be processed, e.g., as obtained from Computed Tomography or Magnetic Resonance Imaging. The ML algorithm may process time varying data, where one dimension stores an image or volume representation at different points in time.

Hereinafter, various examples will be described in the context of a trained ML algorithm configured for processing medical imaging data. However, similar techniques can be readily applied to other kinds and types of semantic context of the imaging data. As a general rule, various kinds and types of medical imaging data can be subject to the techniques described herein. To give a few examples, it would be possible to use magnetic resonance imaging (MRI) imaging data, e.g., raw data in K-space or pre-reconstructed images. Another example would pertain to computed tomography (CT) imaging data, e.g., projection views or pre-reconstructed images. Yet another example would pertain to Positron Emission Tomography (PET) imaging data. Other examples include ultrasound images or a combination of at least two of the medical imaging data outlined above.

As a general rule, various kinds and types of ML algorithms can benefit from the techniques described herein. For instance, it would be possible to use a deep neural network, e.g., a convolutional neural network having one or more convolutional layers performing convolutions between the input data and a kernel. It would also be possible to use a support vector machine, to give just a few examples. A U-net architecture may be used, see, e.g., Ronneberger, O., Fischer, P. and Brox, T., 2015, October. U-net: Convolutional networks for biomedical image segmentation. In International Conference on Medical image computing and computer-assisted intervention (pp. 234-241). Springer, Cham.

In the examples described herein, the ML algorithm can be configured to perform various tasks when processing the medical imaging data. An ML algorithm can also implement combination of such tasks as presented hereinafter. For instance, the ML algorithm could be configured to perform diagnosis of diseases, such as neurological/psychiatric diseases, cancer, hepatitis diseases, and etc. Further, the ML algorithm could be configured to perform a segmentation of medical imaging data. For instance, it would be possible to segment predefined anatomical features. In a further example, the ML algorithm could be configured to perform an object detection. For instance, a bounding box could be drawn around a predefined object that is detected in the medical image data. Predefined objects could be predefined anatomical features, e.g., certain organs or vessels, a tumor site, etc.. It would also be possible to detect anomalies. As will be appreciated from the above, the particular type of the ML algorithm is not germane for the functioning of the techniques described herein. Rather, various kinds and types of ML algorithms can benefit from the techniques described herein, i.e., performance of such trained ML algorithms can be accurately assessed and may thereby be accurately retrained/updated.

Various techniques are based on the finding that the performance of a trained ML algorithm, such as any one of those outlined above, can be automatically assessed by comparing a predicted diagnosis generated by the trained ML algorithm with a validated diagnosis parsed from a validated radiology report.

A radiology report, such as produced by a radiologist, aims at facilitating communication between the radiologist and referring medical doctor. It is part of the patient's permanent health record and interprets the investigation in the clinical context. Although so far, there may not be universally accepted rules for the structure of a radiology report, it can be argued that concise, consistent ordering of the report both reduces variation between reports and makes it easier for referrers who become familiar with the format to assimilate the information. For example, as shown in Table 1, a radiology report may broadly comprise information associated with the following categories: clinical referral, technique, findings, conclusion, and advice. Accordingly, a radiology report may be divided into sections according to different categories. In practice, there may be specific structured radiology reports that may be consistent across one or more particular hospitals/institutions. For example, there may be structured radiology reports applied in a specific country, state, or medical centers of the same university.

**Table 1: Exemplary Categories/Sections of a radiology report**

| Categories/sections of a radiology report | Description |
|---|---|
| Clinical referral | This section may include a brief summary of the reason for referral, summarizing the clinical problem. It may be as concise as possible, but could nevertheless contain all the relevant clinical information. It is important that the clinical history of the patient is provided by the referring physician, in order to enable correct image interpretation. If insufficient clinical information is available for the radiologist to give a full interpretation, this should be stated in the report. When appropriate, the justification of radiation exposure should be given. |
| Technique | This section may include: (a) A concise description of the investigation/procedure performed, with specific mention of any non-standard elements of the investigation, e.g. additional sequences. |
| | (b) A record of contrast medium administration, including route of administration, type, and dose. Any adverse reaction would be recorded and the treatment described. Additional medications that were administered to the patient while in the imaging department (e.g. sedatives) would also be recorded. |
| | (c) A description of technically suboptimal features if they may have an impact on the accuracy of interpretation. (d) Patient radiation dose where applicable. |
| Findings | (a) This section of the report may include a targeted, systematic and comprehensive description of abnormalities and may start with those features that are relevant to the clinical request or suspected pathology. |
| | (b) Observations may be described using accepted imaging terminology, and be as precise as possible, avoiding loose terms such as 'shadowing'. |
| | (c) The description may be specific in giving the dimensions, signal intensity, attenuation, echogenicity or density of abnormalities. Specific positive or negative features which will affect interpretation of the abnormality/-ies, such as clarity of margin, calcification or cavitation may also be described. |
| | (d) The anatomical site of abnormalities may be clearly stated, together with their relationship to other structures where appropriate. |
| | (e) It may be helpful to the referrer and anyone subsequently viewing the images to indicate the relevant images on which the abnormality is best shown and on which the measurements were performed, e.g. series 2, image 12. |
| | (f) Relevant negative findings may be specifically stated. For example, before performing a radiology examination, a checking list may be provided by a referrer to indicate hypothesized abnormalities to be examined, and the radiology report may state the negative findings with respect to the hypothesized abnormalities, i.e., the radiology report may describe not only |
| | abnormalities but also normal anatomical structures. |
| | (g) Incidental findings may be stated and analyzed. |
| | (h) Where previous investigations have been performed, comparison with the current examination may be carried out and described in the report, including the date of the previous examination. The absence of previous imaging may also be recorded if relevant. |
| Conclusion | (a) This section comprises an interpretation of the investigation, taking into account the imaging features, together with relevant clinical information and laboratory findings, to formulate an overall impression. |
| | (b) The aim is to reach a precise diagnosis when possible, or an appropriately ranked differential diagnosis. |
| | (c) Where a differential diagnosis is given, it may be relevant and limited, and the evidence supportive of or against each suggested diagnosis may be explained. |
| | (d) The conclusion relates to the original presentation, e.g. 'no cause for the left chest pain identified'. |
| | (e) Any incidental findings may be clearly described as either relevant or non-significant. |
| | (f) Any adverse event may be restated. |
| Advice | The report may give suggestions for further action to be taken, e.g. referral for an urgent specialist opinion. More commonly, advice will be given on further investigations which will refine the |
| | diagnosis. These suggestions may be carefully considered and not expose the patient to unnecessary further investigations. |

In general, after production of a radiology report by a radiologist, the radiology report may be checked, revised, and signed by at least one clinical referrer and thereby a validated radiology report is generated. The validated radiology report may be stored in a database, such as a picture archiving and communication system (PACS).

According to this disclosure, the (validated) radiology report may be a free-text report or a structured report, e.g., a structured report which is consistent across a particular hospital or institution. Conventional radiology reports are stored as free text, i.e., free-text reports, so information is trapped in the language of the report, making it difficult to find specific details without reading/analyzing the whole text. On the other hand, in structured reports, the information is standardized and presented in a clear, organized format, tracking the attributes of each finding (size, location, etc.) and prompting the radiologist to complete all required fields. Structured reports are time-efficient and may support automatic analysis for research and decision-support. Structured reports may also facilitate retrieval of data by automated or semi-automated methods for the purposes of comparison, audit, and research. Further, structured reports may be structured and/or displayed in a modular format with section headings, contain a consistent ordering of observations in the form of templates or checklists, and use standardized language and lexicon. In other examples, it is also possible to integrate, into structured reports, additional information, such as clinical data, technical parameters, measurements, annotations, and key (relevant) images and an identifier indicating a storage location of medical imaging data associated with the radiology report, giving the potential to reduce ambiguity and increase confidence in the findings.

According to this disclosure, various performance measurements or performance metrics may be applied to evaluate the performance of a trained ML algorithm. The performance measurements may comprise accuracy, sensitivity, and/or specificity. Accuracy estimates the correct classification out of all classifications on a range from 0-1 (the equivalent of 0-100%). Sensitivity explains how many patients with a disease have been correctly identified with this disease (true positive rate), on a range from 0-1 (0-100%). Specificity, in contrast to sensitivity, determines how many patients without a disease have been correctly identified without this disease (true negative rate), on a range from 0-1 (0-100%) . Thus, higher values for accuracy, sensitivity, and specificity indicate a well-trained ML algorithm that provides more accurate results.

As outlined above, by comparing a validated label of at least one diagnosis parsed from a validated radiology report with a prediction of the at least one diagnosis generated by a trained ML algorithm, the performance of the trained ML algorithm can be automatically and precisely evaluated, i.e., there is no need to involve efforts of clinical experts.

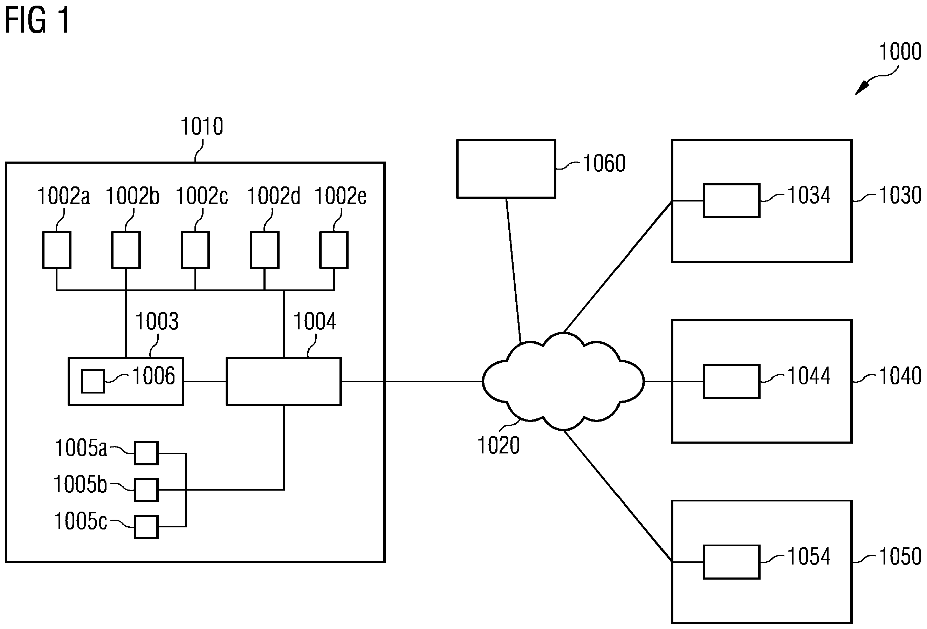

FIG. 1 schematically illustrates details with respect to a system 1000 according to various examples. The system 1000 may include four local networks 1010, 1030, 1040, as well as 1050, which are respectively within four hospitals or institutions, and a(n) external/shared network 1020, such as the internet or a cloud, via which the four local networks 1010, 1030, 1040, as well as 1050 may be communicated with each other. FIG. 1 is only an illustration of one possible example; generally, the number of local networks may be any positive integer, e.g., 1, 2, 3 and so on. The external/shared network 1020 is optional.

Each of the four local networks 1010, 1030, 1040, as well as 1050 may share the same or similar architecture and have the same or similar network elements or devices. For example, the local network 1010 may comprise at least one medical imaging equipment 1002a-1002e, at least one local data repository 1003 comprising a PACS 1006, at least one computing device 1004 connectable to the external/shared network 1020. The computing device 1004 can act as a gateway node to connect to the outside of the local network 1010, e.g., to any network node connectable via the external/shared network 1020. For example, the computing device 1004 can connect, via the external/shared network 1020, to respective computing devices 1034, 1044, and 1054 of respective local networks 1030, 1040, and 1050. Similarly, the respective computing devices 1034, 1044, and 1054 can also act as respective gateway nodes of the respective local networks 1030, 1040, and 1050. The local network 1010 further comprises at least one user terminal 1005a-1005c, which is generally optional. Within the local network 1010, each of the at least one medical equipment 1002a-1002e is respectively connectable to the at least one local data repository 1003 and the at least one computing device 1004 via physical cables or via wireless communication; each of the at least one user terminal 1005a-1005c may be connectable to the at least one computing device 1004 via physical cables or via wireless communication.

According to various examples, the medical imaging equipment 1002a-1002e comprises one or more of an X-ray scanner, a computed tomography scanner, a magnetic resonance imaging scanner, a positron emission tomography scanner, an ultrasound scanner, and so on. A medical imaging examination of a patient can be performed by a radiologist using at least one of the medical imaging equipment 1002a-1002e, with respect to at least one anatomical region of the patient. Medical imaging data are obtained by the medical imaging examination and may be encoded according to a standard, such as the Digital Imaging and Communications in Medicine (DICOM) standard. Other standards, such as JPEG or TIFF, may be used. The (encoded) medical imaging data may be transmitted to the at least one local data repository 1003 and/or the at least one computing device 1004. The (encoded) medical imaging data may be stored in the at least one local data repository 1003 and/or in the at least one computing device 1004.

According to this disclosure, a radiology report may be produced by a radiologist (and/or other medical practitioners) during a medical imaging examination of a patient, such as an ultrasound examination or an angiography examination. Additionally or alternatively, the radiology report may be produced by the radiologist (and/or other medical practitioners) after the medical imaging examination by reviewing/studying the medical imaging data acquired during the medical imaging examination. For example, the radiologist may obtain, via one of the at least one user terminal 1005a-1005c, the medical imaging data from the at least one local data repository 1003 or the at least one computing device 1004, and compile the radiology report. After producing/compiling the radiology report, the radiology report may be also stored in the at least one local data repository 1003 and/or in the at least one computing device 1004. Then, a validated radiology report may be produced by referrers of the radiology report and stored in the at least one local data repository 1003 and/or in the at least one computing device 1004. For example, to obtain the validated radiology report, the referrers may simply add a signature in the radiology report when the referrers agree with the radiology report, or may revise the radiology report and then add a signature in the revised radiology report when the referrers find some mistakes in the radiology report.

According to various examples, the trained ML algorithm outlined above may be executed by the at least one computing device 1004 or by a respective computing device embedded in or connected to a respective medical equipment 1002a-1002e. When a new radiology report is produced, a respective device managing the trained ML algorithm may receive a trigger or notification, e.g., from the computing device 1004 or the local data repository 1003. Alternatively or additionally, the respective device managing the trained ML algorithm may proactively check, from time to time, whether a new radiology report is available. Then, the trained ML algorithm obtain medical imaging data associated with the radiology report and process the medical imaging data to generate a prediction of at least one diagnosis, such as a prediction of a size of a tumor or a prediction of a diameter of an aorta. Before, after, or in parallel with processing the medical imaging data by the trained ML algorithm, the computing device 1004 parses the validated radiology report to obtain a validated label of the at least one diagnosis, such as a validated size of the tumor or a validated diameter of the aorta. Accordingly, the performance of the trained ML algorithm can be determined/evaluated by comparing the prediction of at least one diagnosis and the validated label of the at least one diagnosis.

According to this disclosure, when the performance, e.g., an accuracy, a sensitivity, and/or a specificity, of the trained ML algorithm is lower than a pre-defined threshold, the trained ML algorithm may be retrained based on the medical imaging data and/or validated label of at least one diagnosis parsed from the validated radiology report, e.g., a size of a tumor, existence of a tumor, a diameter of an aorta, using supervised learning, semi-supervised learning, non-supervised learning, or reinforcement learning. I.e., during retraining of the trained ML algorithm, the medical imaging data can be used as input of the trained ML algorithm, and the validated label of at least one diagnosis parsed from the validated radiology report can be used as the ground truth or reference. Additionally or alternatively, if the same trained ML algorithm, i.e., a ML algorithm having the same architecture, is respectively executed by a respective node in the four local networks 1010, 1030, 1040, and 4050, such as computing devices 1004, 1034, 1044, and 1054, federated learning or distributed learning may be utilized to retrain the ML algorithm.

Optionally, the system 1000 may comprise a central computing device 1060 which may be accessible to at least the respective computing devices 1004, 1034, 1044, and 1054 of the respective local networks 1010, 1030, 1040, and 1050. In some examples, each pair of the computing devices 1004, 1034, 1044, and 1054 may not be connectable directly, but can exchange data/information via the central computing device 1060, and thereby security of data generated/stored in the respective local networks 1010, 1030, 1040, and 1050 can be improved, for example by implementing access control techniques at the central computing device 1060. Further, the central computing device 1060 may facilitate a centralized federated learning of a ML algorithm respectively executed by a respective node in the four local networks 1010, 1030, 1040, and 4050.

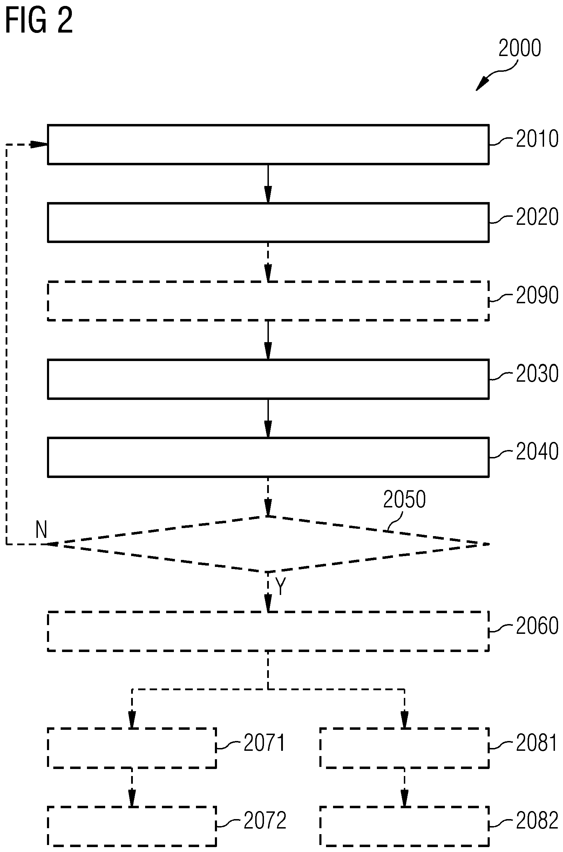

According to the disclosure, a method for determining a performance of a trained ML algorithm is provided. The method may be independently executed by any one of the computing devices 1004, 1034, 1044, and 1054 based on both respective validated radiology reports and respective medical imaging data associated with the respective validated radiology reports which are both produced in respective hospitals or institutions. The method determines the performance of the trained ML algorithm by performing a comparison between a validated label of at least one diagnosis parsed from a validated radiology report and a prediction of the at least one diagnosis generated by the trained machine-learning algorithm based on the medical imaging data associated with the validated radiology report. Details with respect to such a method will be explained in connection with FIG. 2.

FIG. 2 is a flowchart of a method 2000 according to various examples. The method 2000 pertains to determining/evaluating a performance of a trained ML algorithm.

The trained ML algorithm is configured to process medical imaging data and thereby generate a prediction of at least one diagnosis of a patient based on the medical imaging data. The prediction of the at least one diagnosis of the patient is compared with a validated label of the at least one diagnosis of the patient and thereby the performance of the trained ML algorithm is determined based on the comparison. The validated label of the at least one diagnosis of the patient is obtained by parsing a validated radiology report of the patient and the medical imaging data is associated with the validated radiology report.

The method 2000 can be executed by at least one processor upon loading program code. For example, the method 2000 could be executed by a processor of any one of the computing devices 1004, 1034, 1044, and 1054, upon loading program code from a respective memory.

Optional boxes are labeled with dashed lines.

At box 2010, a validated radiology report of a patient and medical imaging data of the patient associated with the validated radiology report are obtained. Box 2010 could include sending control instructions to a medical imaging equipment 1002a-1002e to acquire the medical imaging data and then clinical professionals can produce the validated radiology report interpreting investigations revealed by the medical imaging data. Box 2010 could include loading the validated radiology report and the medical imaging data from a memory of a computing device, such as any one of the computing devices 1004, 1034, 1044, and 1054. Box 2010 could include retrieving the validated radiology report and the medical imaging data from a data repository, such as the data repository 1003 in the local network 1010, or similar data repository in the other local networks 1030, 1040, and 1050.

The medical imaging data could be acquired using multiple configurations of medical imaging equipment 1002a-1002e, or using multiple imaging equipment. For instance, different parameters for the acquisition of the medical imaging data, e.g., exposure time, MRI scanning protocol, CT contrast, etc. could be selected.

At box 2020, the validated radiology report is parsed to obtain a validated label of at least one diagnosis of the patient. Various parsing or syntactic parsing methods could be utilized to parse the validated radiology report to obtain the validated label of the at least one diagnosis of the patient, such as constituency parsing techniques or dependency parsing approaches. The constituency parsing techniques may comprise Cocke-Kasami-Younger algorithm (CKY), transition-based parsing algorithms, and sequence-to-sequence parsing algorithms. The dependency parsing approaches may comprises transition-based, grammar-based, and graph-based algorithms. Alternatively or optionally, other parsing methods may also be used, such as (deep) neural-network-based and transformer-based text mining algorithms.

It is possible to select a parsing or syntactic parsing method based on a type of the validated radiology report. For example, the validated radiology report may comprise a structured report and the parsing of the validated radiology report may comprise extracting the validated label of at least one diagnosis, for example according to a pre-defined format. On the other hand, the validated radiology report may comprise a free-text report and the parsing of the validated radiology report may comprise applying at least one language agnostic and context aware text mining method to the validated radiology report. Optionally or alternatively, The parsing of the validated radiology report may include applying at least one language-specific text mining method to the validated radiology report. For example, the parsing methods may analyze "Findings" and/or "Conclusion" part of the validated radiology report to obtain the validated label of at least one diagnosis. The at least one diagnosis may comprise at least one of the following: at least one abnormality, anatomical site of the at least one abnormality, size of the at least one abnormality, a name of the at least one abnormality.

At an optional box 2090, the trained ML algorithm is selected from a plurality of trained ML algorithms based on the validated label of at least one diagnosis. For example, there may be program codes of the plurality of trained ML algorithms stored in the computing device 1004 of FIG. 1, and each of the plurality of trained ML algorithms may perform a different functionality from the other trained ML algorithms. There could be a mapping which represents relationships between each diagnosis and each of the plurality of trained ML algorithms. For instance, there are four trained ML algorithms for respectively determining which coronary artery suffers from atherosclerosis, where is the plaque causing atherosclerosis, the size of the plaque, the diameter of the section of the coronary artery where the plaque is located. If the validated label of at least one diagnosis indicates "the right coronary artery" or "the left coronary artery", the trained ML algorithm for determining which coronary artery suffers from atherosclerosis is selected.

At box 2030, a prediction of the at least one diagnosis is generated, by a trained ML algorithm and at a computing device, e.g., any one of the computing devices 1004, 1034, 1044, and 1054, based on the medical imaging data. The trained ML algorithm may be selected at box 2090. The trained ML algorithm may be the only ML algorithm executed by the computing device, e.g., a dedicated computing device directly connected to a medical imaging equipment 1002a-1002e. The trained ML algorithm may take a part of the medical imaging data as input at a time point.

At box 2040, a performance of the trained ML algorithm is determined based on a comparison of the validated label of the at least one diagnosis and the prediction of the at least one diagnosis. The performance may be indicated by a deviation between the validated label of the at least one diagnosis of the report and the prediction of the at least one diagnosis of the report. For example, the trained ML algorithm is used to determine a diameter of a section of a coronary artery where a plaque is located, and the validated label of the diameter and the prediction of the diameter are respectively 29 mm and 27 mm.

Accordingly, the performance of the trained ML algorithm is 2 mm. In comparison with 1 mm, 2 mm indicates a lower performance, i.e., the more significant the deviation, the lower the performance.

According to various examples, boxes 2010, 2020, 2030, and 2040 may be iteratively/repeatedly performed based on multiple validated radiology reports. I.e., multiple instances of the performance of the same trained ML algorithm may be generated based on the multiple validated radiology reports, and thereby the performance of the same trained ML algorithm can be determined based on the multiple instances of the performance of the trained ML algorithm in a statistical manner. For example, accuracy, sensitivity, and/or specificity could be determined based on the multiple instances of the performance of the trained ML algorithm. Accordingly, the method 2000 may optionally or additionally include obtaining a further validated radiology report of a further patient and further medical imaging data of the further patient associated with the further validated radiology report; parsing the further validated radiology report to obtain a further validated label of the at least one diagnosis; generating, by the trained machine-learning algorithm and at the computing device 1004, 1034, 1044, 1054, a further prediction of the at least one diagnosis based on the further medical imaging data, and the determining of the performance of the trained machine-learning algorithm is further based on a further comparison of the further validated label of the at least one diagnosis and the further prediction of the at least one diagnosis.

Preferably, the deviation may be visually highlighted to a user by color coding corresponding items in the validated radiology report, flagging a report as being used for machine learning or displaying a list of identified deviations across multiple radiology reports.

According to this disclosure, poor performance of the trained ML algorithm may be caused by vast heterogeneity of the medical imaging data, e.g. variations caused by scan protocols, scanner types, demographics, etc. As such, the trained ML may need considerable re-training efforts to account for conditions and parameters not included in the training pool during development.

Optionally, at box 2050, the performance of the trained ML algorithm is compared with a pre-defined threshold to determine whether the performance of the trained ML algorithm is lower than a pre-defined threshold. Different diagnosis have different pre-defined thresholds. If the performance of the trained ML algorithm is lower than a pre-defined threshold, at box 2060, an update of parameters of the trained ML algorithm is triggered based on the validated label. The update of parameters of the trained ML algorithm can be performed using supervised learning, semi-supervised learning, non-supervised learning, or reinforcement learning. If the performance of the trained ML algorithm is equal to or higher than the pre-defined threshold, box 2010 may be executed.

Such triggering of the update of the parameters can include performing a training process. Such triggering of the update of the parameters could also include requesting another remote device to perform the training process. Sometimes, because the training processes are computationally expensive, the training can be implemented, e.g., at a cloud server. Different scenarios will be explained in further detail below.

According to various examples, updates of the trained ML algorithms, e.g., using retraining techniques, may be done in small incremental developments in a hospital or institution which are usually based on limited feedback from the hospital or institution. Retraining of the trained ML algorithms is a sensitive matter which might improve the algorithm performance towards certain datasets, e.g., medical imaging data acquired in a specific hospital or institution, but it might not be representative of the overall data available in the field, i.e., the ML algorithms may be overfitted. To solve the overfitting issues, medical imaging data acquired in different hospitals/institutions may be shared, however, medical imaging data are usually very big, and consequently, medical imaging data sharing is time-consuming. Further, country-specific regulations may prohibit sharing medical imaging data due to privacy.

Optionally, after performing a local retraining of the trained ML algorithm within a hospital or institution, if the same ML algorithm is also utilized in other hospitals or institutions, for example, executed by at least two of the computing devices 1004, 1034, 1044, and 1054, federated learning may be applied to retrain the trained ML algorithm to mitigate overfitting, to reduce overhead associated with medical imaging data sharing, and to protect privacy. Further, federated learning also can improve performances of the trained ML algorithm running in a small clinic or hospital which provides medical services to a small number of patients by sharing parameters of the trained ML algorithm revised/tailored in big hospitals or university medical centers. Details with respect to updating parameters of the trained ML algorithm using federated learning will be explained in connection with the following two optional examples.

### Example 1:

At box 2071, the updated parameters of the trained machine-learning algorithm are provided to a central computing device, e.g., the central computing device 1060 of FIG. 1.

At box 2072, upon providing the updated parameters, an update of the trained ML algorithm is received from the central computing device. The update of the trained ML algorithm is performed, by the central computing device, using secure aggregation and/or federated averaging based on the updated parameters of the trained ML algorithm and on at least one additional update of the parameters of the trained ML algorithm. The at least one additional update of the parameters may be received by the central computing device from one or more additional computing device, e.g. the computing devices 1034, 1044, and 1054, running the trained ML algorithm.

### Example 2:

At box 2081, at least one additional update of the parameters of the trained ML algorithm is received, at the computing device, e.g., 1004 of FIG. 1, from one or more additional computing devices, e.g., the computing devices 1034, 1044, and 1054 of FIG. 1, running the trained ML algorithm.

At box 2082, an update of the trained ML algorithm is determined, by the computing device, e.g., 1004 of FIG. 1, using secure aggregation and/or federated averaging based on the updated parameters and on the at least one additional update of the parameters.

According to various examples, the medical imaging data of the patient associated with the validated radiology report may be obtained by parsing the validated radiology report. For example, the validated radiology report may comprise an identifier indicating a storage location of medical imaging data associated with the radiology report, and the medical imaging data may be obtained using the identifier.

According to the disclosure, box 2020 can be performed before, after, or in parallel with box 2030.

Once an update of the trained ML algorithm is determined, the updated version of the trained ML algorithm can improve the performance of the trained ML algorithm and thereby facilitate the utilization of the trained ML algorithm in clinical practices. The method 2000 utilizes a validated radiology report to extract a validated label of at least one diagnosis for determining a performance of a trained ML algorithm and optionally for retraining the trained ML algorithm, and thereby the need to tediously transfer, communicate and annotate medical imaging data can be removed. Additionally, a combination of obtaining validated label by parsing a validated radiology report and retraining using federated learning can facilitate a continuous incremental algorithm update. The method 2000 may automatically take all heterogeneous aspects of algorithm deployment in clinical environments into account and thereby refine the trained ML algorithm systematically using medical imaging data representative of heterogeneous clinical environments where these algorithms are deployed.

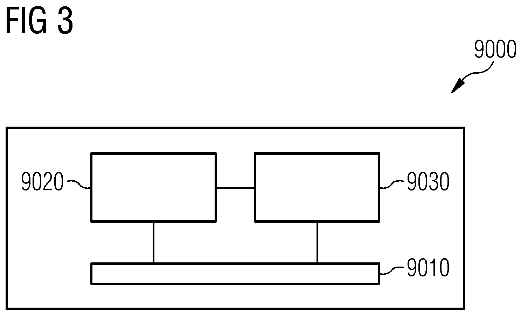

FIG. 3 is a block diagram of a device 9000 according to various examples. The device 9000 may comprise at least one processor 9020, at least one memory 9030, and at least one input/output interface 9010. The at least one processor 9020 is configured to load program code from the at least one memory 9030 and execute the program code. Upon executing the program code, the at least one processor 9020 performs the method 2000.

Additionally or alternatively, the device 9000 may be embedded in any one of the medical imaging equipment 1002a-1002e of FIG. 1, and thereby the medical imaging equipment may be also configured to perform the method 2000.

Summarizing, techniques have been described that facilitate automatically determining a performance of a trained ML algorithm and thereby retraining the trained ML algorithm when the performance is poor. Validated radiology reports are utilized to extract a validated label of at least one diagnosis for determining a performance of a trained ML algorithm and optionally for retraining the trained ML algorithm, and thereby the need to tediously transfer, communicate and annotate medical imaging data can be removed. Additionally, a combination of obtaining validated label by parsing a validated radiology report and retraining using federated learning can facilitate a continuous incremental algorithm update. Such techniques can automatically take all heterogeneous aspects of algorithm deployment in clinical environments into account and thereby refine the trained ML algorithm systematically using medical imaging data representative of heterogeneous clinical environments where these algorithms are deployed.

Although the invention has been shown and described with respect to certain preferred embodiments, equivalents and modifications will occur to others skilled in the art upon the reading and understanding of the specification. The present invention includes all such equivalents and modifications and is limited only by the scope of the appended claims.

## Claims

1. A computer-implemented method (2000), the method (2000) comprising:
- obtaining (2010) a validated radiology report of a patient and medical imaging data of the patient associated with the validated radiology report;
- parsing (2020) the validated radiology report to obtain a validated label of at least one diagnosis;
- generating (2030), by a trained machine-learning algorithm and at a computing device (1004, 1034, 1044, 1054), a prediction of the at least one diagnosis based on the medical imaging data; and
- determining (2040) a performance of the trained machine-learning algorithm based on a comparison of the validated label of the at least one diagnosis and the prediction of the at least one diagnosis.

2. The computer-implemented method (2000) of claim 1, the method (2000) further comprising:
- triggering (2060) an update of parameters of the trained machine-learning algorithm based on the validated label, when the performance of the trained machine-learning algorithm is lower than a pre-defined threshold.

3. The computer-implemented method (2000) of claim 2, the method (2000) further comprising:
- providing (2071), to a central computing device (1060), the updated parameters of the trained machine-learning algorithm; and
- upon providing the updated parameters, receiving (2072), from the central computing device (1060), an update of the trained machine-learning algorithm.

4. The computer-implemented method (2000) of claim 3,
wherein the update of the trained machine-learning algorithm is performed, by the central computing device (1060), using secure aggregation and/or federated averaging based on the updated parameters of the trained machine-learning algorithm and on at least one additional update of the parameters of the trained machine-learning algorithm, the at least one additional update of the parameters being received by the central computing device (1060) from one or more additional computing device (1004, 1034, 1044, 1054) running the trained machine-learning algorithm.

5. The computer-implemented method (2000) of claim 2, the method (2000) further comprising:
- receiving (2081), at the computing device (1004, 1034, 1044, 1054) and from one or more additional computing device (1004, 1034, 1044, 1054) running the trained machine-learning algorithm, at least one additional update of the parameters of the trained machine-learning algorithm; and
- determining (2082) an update of the trained machine-learning algorithm using secure aggregation and/or federated averaging based on the updated parameters and on the at least one additional update of the parameters.

6. The computer-implemented method (2000) of any one of the preceding claims, the method (2000) further comprising:
- selecting (2090) the trained machine-learning algorithm from a plurality of trained machine-learning algorithms based on the validated label of at least one diagnosis.

7. The computer-implemented method (2000) of any one of the preceding claims,
wherein the validated radiology report comprises a structured report and said parsing of the validated radiology report comprises extracting the validated label of at least one diagnosis.

8. The computer-implemented method (2000) of any one of claims 1-6,
wherein the validated radiology report comprises a free-text report and said parsing of the validated radiology report comprises
applying at least one language agnostic and context aware text mining method to the validated radiology report; Or
applying at least one language-specific text mining method to the validated radiology report.

9. The computer-implemented method (2000) of any one of the preceding claims,
wherein the performance is indicated by a deviation between the validated label of the at least one diagnosis of the report and the prediction of the at least one diagnosis of the report.

10. The computer-implemented method (2000) of any one of the preceding claims,
wherein the at least one diagnosis comprises at least one of the following: anatomical site of at least one abnormality, a size of the at least one abnormality, a name of the at least one abnormality.

11. The computer-implemented method (2000) of any one of the preceding claims, the method (2000) further comprising:
- obtaining a further validated radiology report of a further patient and further medical imaging data of the further patient associated with the further validated radiology report;
- parsing the further validated radiology report to obtain a further validated label of the at least one diagnosis;
- generating, by the trained machine-learning algorithm and at the computing device (1004, 1034, 1044, 1054), a further prediction of the at least one diagnosis based on the further medical imaging data;
wherein the determining of the performance of the trained machine-learning algorithm is further based on a further comparison of the further validated label of the at least one diagnosis and the further prediction of the at least one diagnosis.

12. A device (9000), the device (9000) comprising at least one processor (9020) and the at least one processor (9020) being configured to:
- obtain (2010) a validated radiology report of a patient and medical imaging data of the patient associated with the validated radiology report;
- parse (2020) the validated radiology report to obtain a validated label of at least one diagnosis;
- generate (2030), by a trained machine-learning algorithm and at a computing device, a prediction of the at least one diagnosis based on the medical imaging data; and
- determine (2040) a performance of the trained machine-learning algorithm based on a comparison of the validated label of the at least one diagnosis and the prediction of the at least one diagnosis.

13. The device (9000) of claim 12, the device further configured to perform the method of any one of claims 1-11.

14. A medical imaging equipment (1002a-1002e), the medical imaging equipment (1002a-1002e) comprising the device of claim 12 or 13.

15. Computer program comprising instructions which, when the program is executed by a processor (9020), cause the processor (9020) to carry out the computer implemented method of one of the claims 1 to 11.

16. Computer-readable storage medium having stored thereon the computer program of claim 15.
